# NOUVEAU FASCICULE DE BREVET EUROPEEN

(11) **EP 2 854 803 B2**
(45) Date de publication et mention de la décision concernant l'opposition: **24.08.2022**
(45) Mention de la délivrance du brevet: 27.06.2018
(21) Numéro de dépôt: 13728696.9
(22) Date de dépôt: 30.05.2013
(51) Int. Cl.: A61K 31/40, A61P 17/02, A61P 17/10, A61P 17/12, A61P 17/16, A61K 8/34, A61K 8/49, A61K 8/67, A61P 19/00, A61K 8/81, A61K 8/04

(54) **COMPOSITIONS TOPIQUES SOUS FORME DE GEL CONTENANT UN RÉTINOÏDE PARTICULIER SOLUBILISÉ**
TOPISCHE GEL ZUSAMMENSETZUNG, DIE EIN SPEZIFISCHE SOLUBILIZIERTE RETINOID ENTHÄLT
TOPICAL GEL COMPOSITION COMPRISING A SOLUBILIZED SPECIFIC RETINOID

(30) Priorité: 01.06.2012 FR 1255092; 01.06.2012 US 201261654702 P
(43) Date de publication de la demande: 08.04.2015
(73) Titulaire: Galderma Research & Development, 06410 Biot (FR)
(72) Inventeur: DUPRAT, Agnès, 06250 Mougins (FR); MALLARD, Claire, 06250 Mougins (FR)
(74) Mandataire: Aera A/S
(86) Numéro de dépôt international: PCT/EP2013/061200
(87) Numéro de publication internationale: WO 2013/178759

(56) Documents cités:
- WO-A1-2006/066978
- WO-A1-2006/066978
- WO-A1-2008/148968
- FR-A1- 2 915 682
- Extract from www.clinicaltrials.Gov/ct2/show/NCT0255678 8

## Description

L'invention se rapporte à une composition de type gel aqueux, de préférence hydroglycolique, et qui comprend, dans un milieu physiologiquement acceptable, au moins un rétinoïde, l'acide 3"-tert-butyl-4'-(2-hydroxy-ethoxy)-4"-pyrrolidin-1-yl-[1,1';3',1"]-terphenyl-4-carboxylique.

Ces composés, décrits dans le brevet EP1831149, sont de puissants rétinoïdes modulateurs du récepteur nucléaires de l'acide rétinoïque (RAR). Plus spécifiquement du sous-type gamme de ce récepteur (RARy).

Les récepteurs RARs activent la transcription en se liant à des éléments de séquences d'ADN, appelés les éléments de réponse des RAR Element (RARE), sous forme d'un hétérodimère avec les récepteurs X des rétinoïdes (appelés les RXRs).

Trois sous-types de RARs humains ont été identifiés et décrits : les RARα, RARβ et RARγ.

Les récepteurs RAR gamma étant situés dans l'épiderme, il est important que la libération du composé décrit par l'acide 3"-tert-butyl-4'-(2-hydroxy-ethoxy)-4"-pyrrolidin-1-yl-[1,1';3',1"]-terphenyl-4-carboxylique s'effectue dans cette partie de la peau pour avoir une efficacité clinique.

Or l'application topique de rétinoïdes peut entraîner une irritation de la peau, une sécheresse et un érythème. De nombreux articles décrivent cet effet irritant comme les articles de Stücker & al. Skin Res Technol. 2002 May;8(2):133-40 ou de Thielitz & al. Am J Clin Dermatol. 2008;9(6):369-81.

Pour obtenir des préparations topiques à usage pharmaceutique contenant des rétinoïdes, de nombreuses techniques sont utilisées, en particulier des émulsions comme par exemple le brevet EP-826366 qui décrit des émulsions pouvant contenir des rétinoïdes ou encore le brevet EP-989846 qui décrit des émulsions contenant des rétinoïdes et au moins un émulsionnant.

Or les émulsionnant font partie de la famille chimique des molécules amphiphiles qui sont souvent irritantes. Les compositions sans émulsionnant sont de ce fait moins irritantes que celles en contenant.

Le fait de ne pas utiliser d'émulsionnant dans les compositions contenant des rétinoïdes permettrait donc de limiter l'irritation cutanée due à la présence de cette classe de molécules.

L'art antérieur décrit des émulsions H/E avec ou sans émulsionnant. On peut citer notamment le brevet US 5,851,538 qui décrit des formulations avec ou sans émulsionnant avec des microsphères poreuses contenant un réseau pratiquement continu de pores ouverts vers l'extérieur et comprenant des rétinoïdes.

Il existe par conséquent un besoin d'avoir des compositions pharmaceutiques stables et bien tolérées, contenant le composé décrit par l'acide 3"-tert-butyl-4'-(2-hydroxy-ethoxy)-4"-pyrrolidin-1-yl-[1,1';3',1"]-terphenyl-4-carboxylique.

Le développement de formes galéniques de type gel hydroglycolique qui ne contiennent en général pas d'émulsionnant apportera donc un avantage en terme de tolérance cutanée.

Selon la FDA, un gel est une forme galénique semi-solide qui contient un agent gélifiant conférant de la consistance à une solution ou une dispersion colloïdale. Un gel hydroglycolique est donc un gel selon cette définition dont la phase gélifiée contient de l'eau et un ou plusieurs glycols.

Un premier objet selon l'invention concerne une composition pharmaceutique comprenant au moins un composé, caractérisée en ce que le composé est l'acide 3"-tert-butyl-4'-(2-hydroxy-ethoxy)-4"-pyrrolidin-1-yl-[1,1';3',1"]-terphenyl-4-carboxylique;
de l'eau, au moins un agent gélifiant, au moins un solvant hydrophile du composé d'acide 3"-tert-butyl-4'-(2-hydroxy-ethoxy)-4"-pyrrolidin-1-yl-[1,1';3',1"]-terphenyl-4-carboxylique, choisi parmi le phénoxyéthanol, l'éthanol et un mélange de phénoxyéthanol et d'éthanol, et au moins un co-solvant hydrophile de l'acide 3"-tert-butyl-4'-(2-hydroxy-ethoxy)-4"-pyrrolidin-1-yl-[1,1';3',1"]-terphenyl-4-carboxylique, caractérisée en ce que le co-solvant est choisi parmi le propylène glycol, le dipropylène glycol et leurs mélanges.

Préférentiellement la composition est un gel hydroglycolique caractérisée en ce que l'acide 3"-tert-butyl-4'-(2-hydroxy-ethoxy)-4"-pyrrolidin-1-yl-[1,1';3',1"]-terphenyl-4-carboxylique est solubilisé.

Un second objet selon l'invention concerne une composition pharmaceutique telle que décrite ci-dessus pour son utilisation en tant que médicament.

Un troisième objet selon l'invention concerne une composition pharmaceutique telle que décrite ci-dessus pour son utilisation dans le traitement des pathologies telles que :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamen-teuse ou professionnelle ;
2) les troubles de la kératinisation, notam mentlesichtyoses, les états ichtyosiformes, l'ichtyoselamellaire, la maladie de Darrier, les kératodermies palmoplantaires,les leucoplasies, le pityriasis rubrapilaireetles états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pig-mentations et les rides ;
5) Toute condition liée à des proliférations dermiques ou épidermiques bénignes, qu'elles soient ou non d'origine virale telles que verrues vulgaires, les verrues planes, le molluscum contagiosum et l'épidermodysplasie verruci-forme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tek que le lymphome T;

L'invention sera décrite plus en détail dans la description et les exemples qui suivent, ainsi que dans les figures annexées dans lesquelles :

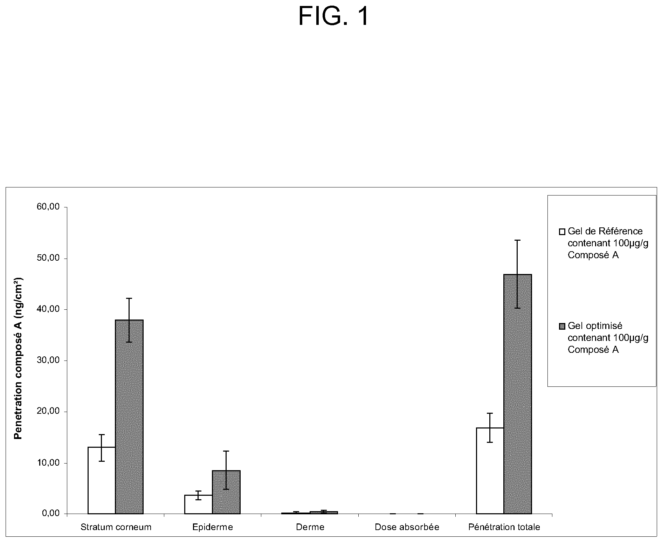

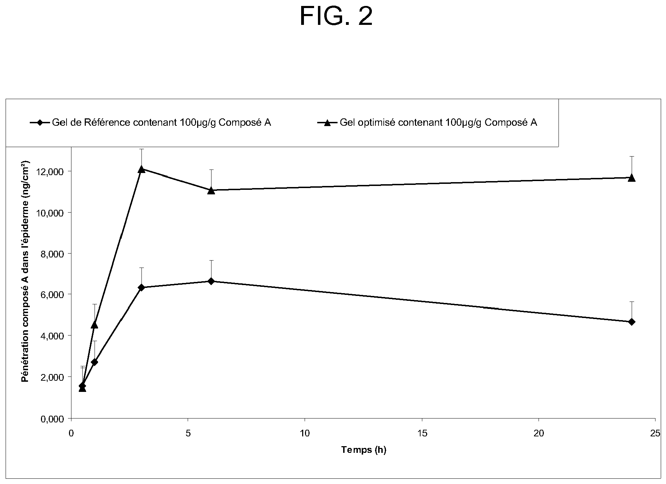

Les Figures 1 et 2 présentent les résultats d'une étude de cinétique de pénétration dans l'épiderme d'une composition selon l'invention et d'un gel de référence.

### Description détaillée de l'invention :

A des fins de facilité de lecture, on considèrera le composé A dans la suite du texte comme étant décrit comme il suit :

Composé A: comme étant l'acide 3"-tert-butyl-4'-(2-hydroxy-ethoxy)-4"-pyrrolidin-1-yl-[1,1';3',1"]-terphenyl-4-carboxylique

Au vu des caractéristiques physico-chimiques de l'actif, la Demanderesse a dû faire face à un certain nombre de contraintes de mise en œuvre du composé A. Ce composé:
- est insoluble dans l'eau,
- est particulièrement soluble dans deux solvants déterminés à partir des études de pré-formulation telles que décrites en exemple 1,
- est stable dans seulement deux co-solvants du composé A, déterminés à partir des études de pré-formulation telles que décrites en exemple 1.

De plus les concentrations maximum des solvants principaux dans les compositions pharmaceutiques sont de préférence limitées pour optimiser la tolérance. Ainsi, la composition selon l'invention contient de préférence une quantité maximale de 1% en poids de phénoxyéthanol. Elle contient également de préférence une quantité maximale de 30% en poids d'éthanol.

Un premier objet selon la présente invention concerne des compositions pharmaceutiques contenant au moins un actif qui est un composé d'acide 3"-tert-butyl-4'-(2-hydroxy-ethoxy)-4"-pyrrolidin-1-yl-[1,1';3',1"]-terphenyl-4-carboxylique, ces compositions se présentant sous la forme de gel aqueux, de préférence hydroglycolique, et dans lesquelles l'actif est solubilisé.

De tels gels présentent une bonne stabilité physique et chimique, une vitesse de pénétration rapide et un niveau de pénétration élevé dans l'épiderme.

Selon la FDA, un gel est une forme galénique semi-solide qui contient un agent gélifiant conférant de la consistance à une solution ou une dispersion colloïdale. Un gel hydroglycolique est donc un gel selon cette définition dont la phase gélifiée contient de l'eau et un ou plusieurs glycols.

Dans l'invention, les compositions contiennent l'actif décrit par l'acide 3"-tert-butyl-4'-(2-hydroxy-ethoxy)-4"-pyrrolidin-1-yl-[1,1';3',1"]-terphenyl-4-carboxylique à des concentrations allant de préférence de 0.00001% à 1% en poids, plus préférentiellement de 0.0001 à 0.1 % en poids et plus préférentiellement de 0.001 à 0.1 % en poids par rapport au poids total de la composition.

Dans l'invention, les compositions contiennent au moins un solvant hydrophile du composé A choisi parmi le phénoxyéthanol, l'éthanol et un mélange de phénoxyéthanol et d'éthanol.

Par solvant on entend un liquide qui a la propriété de dissoudre, de diluer ou d'extraire des substances sans provoquer de modification chimique de ces substances et sans lui-même se modifier.

Selon l'invention, le solvant hydrophile est un tel liquide, dans lequel le composé d'acide 3"-tert-butyl-4'-(2-hydroxy-ethoxy)-4"-pyrrolidin-1-yl-[1,1';3',1"]-terphenyl-4-carboxylique (et plus particulièrement le composé A) présentent une solubilité, à température ambiante et pression atmosphérique, supérieure ou égale à 0,1% en poids.

Le solvant comprend préférentiellement le phénoxyéthanol vendu par exemple sous le nom de phénoxétol par Clariant, et pouvant être utilisé en une teneur allant de 0.2 à 5% en poids et plus préférentiellement de 0.5 à 2% en poids par rapport au poids total de la composition. Le solvant peut également comprendre de l'éthanol pouvant être utilisé de 5 à 50% en ppids et préférentiellement de 15 à 30% en poids par rapport au poids total de la composition.

Dans l'invention, les compositions contiennent au moins un co-solvant, le co-solvant étant choisi parmi le propylène glycol, le dipropylène glycol et leurs mélanges.

Par co-solvant on entend une substance ayant un rôle de solvant en association avec une autre substance.

Au vu des résultats de pré-formulation (Exemple 1) le co-solvant est choisi parmi le monopropylène glycol et le dipropylène glycol et peut être utilisé à des teneurs allant de 2 à 50% en poids et préférentiellement de 10 à 40% en poids par rapport au poids total de la composition.

Dans l'invention, les compositions contiennent au moins un agent gélifiant..

Par agent gélifiant, on entend un composé polymère apte à conférer à la composition la texture d'un gel.

Le ou les agents gélifiants peuvent être notamment choisis parmi les polymères d'origine végétale, les gommes, les pectines, la cellulose et ses dérivés, les polymères d'origine microbiologiques tel que la gomme xanthane, et les polymères gélifiants d'origine synthétique.

A titre d'exemple non limitatif d'agents gélifiants pouvant entrer dans les compositions, on peut citer l'Acrylates/C10-30 Alkyl Acrylate Crosspolymer vendu sous le nom de Pemulen TR-1 ou Pemulen TR-2 par la société Lubrizol, les gélifiants de la famille des polyacrylamides comme le mélange Sodium acrylamide/acryloyldimethyltaurate copolymer / isohexadecane / polysorbate 80 vendu sous le nom de Simulgel 600PHA par la société SEPPIC, le mélange polyacrylamide/isoparaffine C13-14/Iaureth-7 vendu sous le nom de Sepigel 305 par la société SEPPIC,

les carbomers vendus sous le nom d'Ultrez 20^{®}, d'Ultrez 10^{®}, de Carbopol 1382^{®} ou de Carbopol ETD2020NF^{®}, de Carbopol 981 ou encore Carbopol 980 par la Société Lubrizol, les polysaccharides avec à titre d'exemples non limitatifs la gomme de xanthane telle que le Xantural180^{®} vendu par la société Kelco, la gellan gum vendu sous le nom de Kelcogel par la société Kelco, la gomme guar, la cellulose et ses dérivés tel que la microcristalline cellulose et carboxymethyl cellulose de sodium vendue sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250^{®} par la société Ashland, la sodium carboxymethylcellulose en particulier la Blanose cellulose gum 7F vendu par la société Ashland, la famille des aluminium magnésium silicates tel que le Veegum K vendu par la société Vanderbilt, la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges, la famille des carraghénanes en particulier réparties sous quatre grandes familles : κ, λ, β, ω tel que les Viscarin^{®} et les Gelcarin^{®} commercialisés par la société IMCD

De façon préférentielle un agent gélifiant de type polyacrylamide comme le Simulgel 600 PHA^{®} est utilisé, à des concentrations allant de 0,005 à 5 % en poids et préférentiellement, allant de 1% à 4% en poids, seul ou associé à au moins un des gélifiants cités ci-dessus à des concentrations allant de 0,005 à 3 % en poids.

Dans l'invention, les compositions peuvent contenir des additifs parmi lesquels on peut citer les catégories suivantes (utilisés seuls ou en combinaison):
- Des agents conservateurs, tels le methyl paraben, le propyl paraben, le chlorure de benzalkonium, le phénoxyéthanol vendu sous le nom de phénoxétol par Clariant, l'alcool benzylique vendu sous le nom d'alcool benzylique par Merck, le Sorbate de potassium vendu sous le nom de Sorbate de potassium par VWR, l'acide benzoique vendu sous le nom Acide benzoïque par VWR, le 2-Bromo-2-Nitropropane-1,3-Diol vendu sous le nom de Bronopol par Jan Dekker International la chlorhexidine vendu sous le nom de Chlorexidine digluconate 20% solution par Arnaud Pharmacie, le chlorocrésol et ses dérivés, le sodium benzoate vendu sous le nom de Probenz SP par Unipex, l'alcool éthylique et la diazolidinylurée. Ces conservateurs peuvent être utilisés seul ou en association afin de protéger efficacement les formules contre toute contamination bactérienne.
- Des agents permettant d'améliorer les propriétés des formules à l'application comme la Cyclomethicone (St-Cyclomethicone 5NF) ou la Dimethicone (Q7 9120 silicon fluid de viscosité de 20 cst à 12500 cst de Dow Corning).
- Des agents chélatant comme l'EDTA (ethylenediaminetetraacetic acid) et ses dérivés ou sels, la dihydroglycerine, les acides citriques et tartriques, la gluconolactone vendu sous le nom glucono-delta-lactone SG par Jungbunzlauer ou des mélanges de ceux-ci.
- Des agents antioxydants tel que la vitamine E et ses dérivés, comme le DL alpha Tocophérol ou l'acétate de tocophérol de Roche, la vitamine C et ses dérivés, comme l'Ascorbyl Palmitate de Roche, le Butylhydroxytoluene vendu sous le nom de Nipanox BHT par Clariant.
- Des agents apaisants et/ou anti-irritants tels que le PPG-12/SMDI copolymer vendu par la société Bertek pharmaceuticals sous le nom commercial de Polyolprepolymer-2, l'acide glycyrrhetinique ou ses dérivés comme par exemple l'Enoxolone vendu par la société BASF, l'acide hyaluronique tel quel ou sous sa forme hyaluronate de sodium vendu sous le nom commercial de HYAL. NA PWD PH 15-51-45 par la société Contipro, l'allantoine vendue sous le nom de RONACARE ALLANTOINE par MERCK.
- Tout autre additif usuellement utilisé dans le domaine pharmaceutique et cosmétique permettant de conférer à la dite préparation des propriétés spécifiques.

La composition selon l'invention présente avantageusement la composition générale suivante, les pourcentages étant exprimés en poids par rapport au poids total de la composition :
- de 0,00001% à 1% en poids, préférentiellement de 0.0001 à 0.1 % en poids, et de manière plus préférée de 0,001 à 0.1 % en poids de composé A,
- de l'eau,
- de 0,005 à 10 % en poids et préférentiellement de 1 à 4% en poids d'agent gélifiant,
- de 0,2 à 50% en poids et préférentiellement de 0,5 à 30% en poids de solvant hydrophile A choisi parmi le phénoxyéthanol, l'éthanol et un mélange de phénoxyéthanol et d'éthanol,
- de 2 à 50% en poids et préférentiellement de 10 à 40% en poids de co-solvant,
- en option, de 0 à 15% en poids et préférentiellement de 0,1 à 10% en poids d'un ou plusieurs additifs.

Un autre objet selon l'invention concerne un procédé de préparation d'une composition pharmaceutique décrite telle que précédemment et comprenant les étapes suivantes :
A) Préparation de la phase aqueuse : solubiliser les additifs hydrophiles dans l'eau sous agitation.
B) Préparation de la phase active :
   Solubiliser sous agitation le composé A dans le solvant hydrophile (par exemple le phénoxyéthanol), si nécessaire à chaud.
   A température ambiante, ajouter le ou les cosolvants (par exemple l'éthanol et le propylène glycol).
C) Mélange des deux phases :
   Gélifier la phase aqueuse en ajoutant l'agent épaississant (par exemple le Simulgel) 600Pha sous agitation, puis ajouter ensuite la phase active.
Une fois ce mélange homogène, ajouter la cyclomethicone-5 (si présente dans la formule) sous agitation.

### Exemples :

### Exemple 1- Pré-formulation

Afin de réaliser un gel hydro-glycolique contenant une quantité d'éthanol inférieure à 30%, dans lequel le composé A est solubilisé, des études de préformulations ont été réalisées afin de mettre en évidence les excipients permettant une bonne solubilisation ainsi qu'une bonne stabilité de l'actif.

### - (1) Liste des excipients ou de mélange d'excipients hydrophiles dans lesquels la solubilité maximale a été déterminée par HPLC :

| **Nom commercial** | **Nom INCI** | **Solubilité maximale en %** |
|---|---|---|
| Pharmasolve | Methyl Pyrolidone | 4,84 |
| Transcutol | Ethoxy diglycol | 4,17 |
| Alcool Benzylique | Benzoyl Alcool | 2,3880 |
| PEG 400 | Peg-8 | 1,52 |
| Phénoxétol | Phenoxyethanol | 1,957 |
| Ethanol 95-96% | Ethanol | 0,92 |
| Hydrolite 5P | Pentylene glycol | 0,482 |
| Arlasolve DMI | Dimethyl Isosorbide | 0,4 |
| Hexylene Glycol | Hexylene Glycol | 0,4 |
| Dipropylène Glycol Care | Dipropylene Glycol | 0,4 |
| Propylène Glycol | Propylene Glycol | 0,111 |
| Glycérine | Glycerin | 0,002 |

Cette étude montre que 6 solvants peuvent être considérés comme des « solvants principaux » du composé A (Pharmasolve, Transcutol, alcool benzylique, PEG 400, Phenoxetol et éthanol), les autres pouvant être utilisés comme « co-solvants » mis à part la glycérine dans laquelle le composé A est pratiquement insoluble.

### - (2) Stabilité du composé A dans ses solvants principaux déterminée par HPLC

| **Excipients** | | **Composé A** | **Résultats de stabilité** |
|---|---|---|---|
| **Nom commercial** | **Nom INCI** | **%** | |
| Pharmasolve | Methyl Pyrolidone | 0,03% | Instable |
| Transcutol | Ethoxy diglycol | 0,03% | Instable |
| Alcool Benzylique | Benzoyl Alcool | 0,01% | Instable |
| PEG 400 | Poly Ethylene Glycol | 0,3% | Instable |
| Phénoxétol | Phenoxyethanol | 0,05% | Stable |
| Ethanol 95-96% | Ethanol | 0,005% | Stable |

Ces études de stabilité montrent que parmi les « solvants principaux » identifiés, le composé A est stable uniquement dans le phénoxyéthanol et l'éthanol.

### - (3) Stabilité du composé A dans ses co-solvants déterminée par HPLC

| **Excipients** | | **COMPOSÉ A** | **Résultats de stabilité** |
|---|---|---|---|
| **Nom commercial** | **Nom INCI** | **%** | |
| Hydrolite 5P | Pentylene glycol | 0,03% | Instable |
| Arlasolve DMI | Dimethyl Isosorbide | 0,03% | Instable |
| Hexylene Glycol | Hexylene Glycol | 0,001% | Instable |
| Dipropylene Glycol Care | Dipropylene Glycol | 0,3% | Stable |
| Propylène Glycol | Propylene Glycol | 0,05% | Stable |

Ces études de stabilité montrent que parmi les « co-solvants » identifiés, le composé A est stable uniquement dans le dipropylène glycol et le propylène glycol.

### Exemple 2 : Formulations

Dans les exemples suivants, les formules réalisées sont caractérisées à T0. La stabilité physique et chimique des formulations est réalisée après conservation à température ambiante (TA) et +40°C après T+1Mois et/ou T+2Mois ou T+3Mois ou T+6Mois. Le matériel et les méthodes utilisés pour ces caractérisations sont décrits ci-dessous.

### -Dosage chimique du composé A:

- Matériel: HPLC
- Expression des résultats: le titre de l'actif est exprimé en % relatif par rapport au % initial effectué à T0. Les limites fixées pour une bonne stabilité sont 95%-105%

### -Observation macroscopique:

- L'observation macroscopique permet de garantir l'intégrité physique des produits à T0 et après stabilité.

### -Observation microscopique:

- L'observation microscopique permet d'évaluer la bonne solubilisation du COMPOSÉ A dès T0, la non recristallisation au cours du temps ainsi que la taille des globules de la phase huileuse.
- Matériel: Microscope AXIO ZEISS

### -pH:

- Matériel: pH mètre METTLER TOLEDO Seven Multi
- Méthode : Mesures effectuées à température ambiante après stabilisation 24h dans une enceinte à 25°C de tous les échantillons.

### -Viscosité:

- La mesure de la viscosité permet d'évaluer la consistance des formules réalisées.
- Matériel: Brookfield RV DVII + Pro
- Méthode: Mesures effectuées à température ambiante après stabilisation 24h dans une enceinte à 25°C de tous les échantillons. La valeur est lue après 1 minute. Le choix du mobile et de la vitesse seront décrits dans chaque exemple de composition. Les valeurs obtenues sont exprimées en centipoises (Cps)

### -Centrifugation:

- La centrifugation permet d'évaluer la résistance des formules à une contrainte mécanique.
- Matériel: Galaxy 14D VWR
- Méthode: 30 minutes à 5000 rpm
- Un résultat conforme signifie qu'il n'y a ni séparation de phases, ni exudat.

### Formule n°1

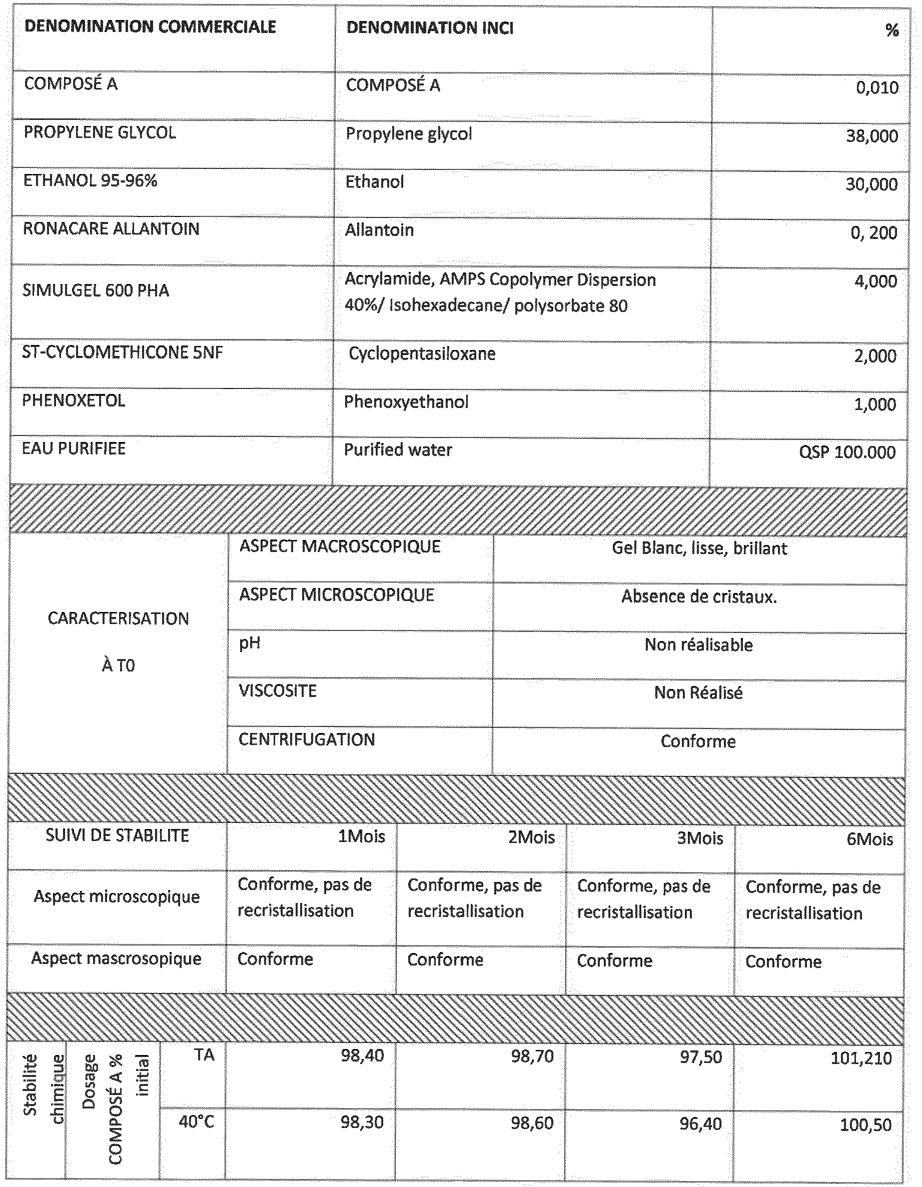

Ces résultats montrent une bonne stabilité physique et chimique de l'actif et de la composition dans son ensemble dans le temps.

### Formule n°2

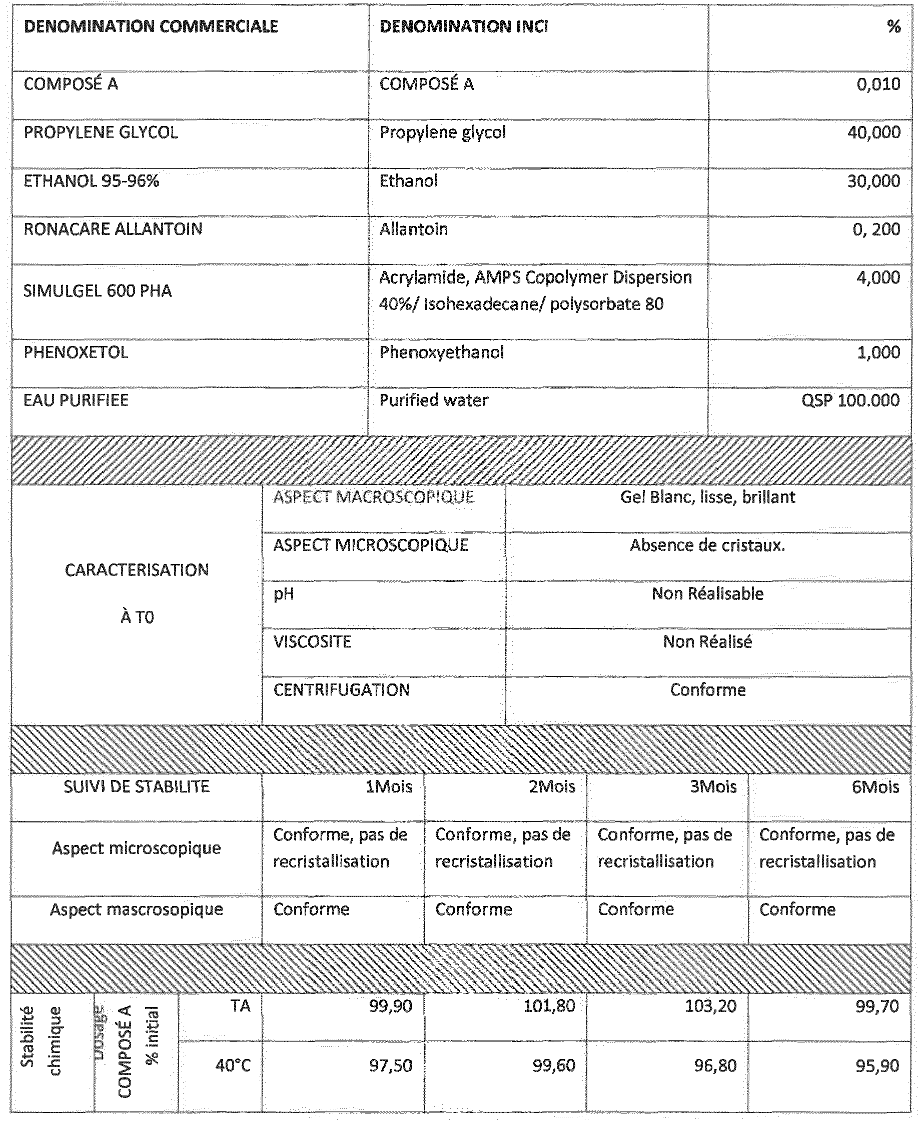

Ces résultats montrent une bonne stabilité physique et chimique de l'actif et de la composition dans son ensemble dans le temps.

### Formule n°3

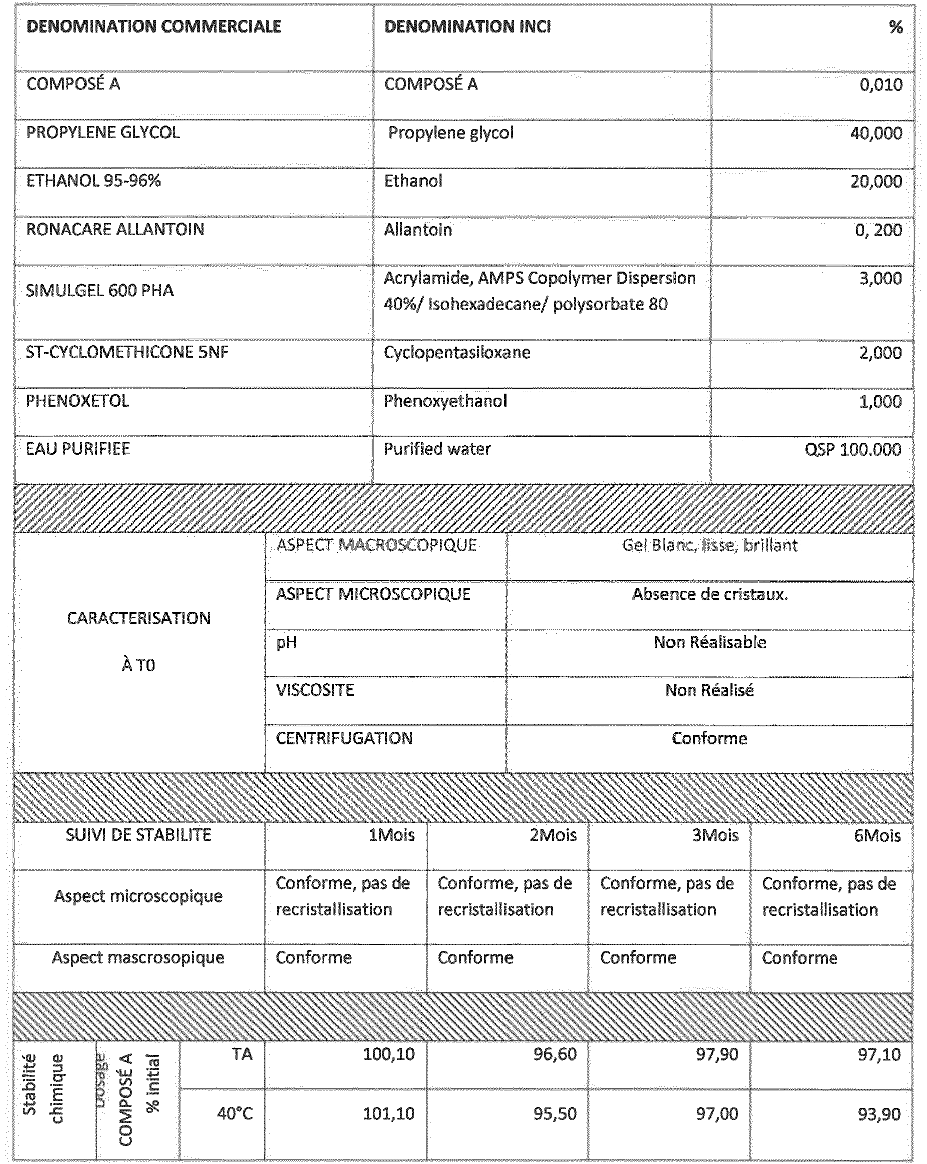

Ces résultats montrent une bonne stabilité physique et chimique de l'actif et de la composition dans son ensemble dans le temps.

### Formule n°4

| **DENOMINATION COMMERCIALE** | **DENOMINATION INCI** | **%** |
|---|---|---|
| COMPOSÉ A | COMPOSÉ A | 0,010 |
| DIPROPYLENE GLYCOL CARE | Dipropylene glycol | 40,000 |
| ETHANOL 95-96% | Ethanol | 20,000 |
| RONACARE ALLANTOIN | Allantoin | 0,200 |
| SIMULGEL 600 PHA | Acrylamide, AMPS Copolymer Dispersion 40%/ Isohexadecane/ polysorbate 80 | 3,000 |
| ST-CYCLOMETHICONE 5NF | Cyclopentasiloxane | 2,000 |
| PHENOXETOL | Phenoxyethanol | 1,000 |
| EAU PURIFIEE | Purified water | QSP 100.000 |

### Formule n°5:

| **DENOMINATION COMMERCIALE** | **DENOMINATION INCI** | **%** |
|---|---|---|
| COMPOSÉ A | COMPOSÉ A | 0,020 |
| DIPROPYLENE GLYCOL CARE | Dipropylene glycol | 40,000 |
| ETHANOL 95-96% | Ethanol | 20,000 |
| RONACARE ALLANTOIN | Allantoin | 0,200 |
| SIMULGEL 600 PHA | Acrylamide, AMPS Copolymer Dispersion 40%/ Isohexadecane/ polysorbate 80 | 3,000 |
| PHENOXETOL | Phenoxyethanol | 2,000 |
| EAU PURIFIEE | Purified water | QSP 100.000 |

### Formule n°6:

| **DENOMINATION COMMERCIALE** | **DENOMINATION INCI** | **%** |
|---|---|---|
| COMPOSÉ A | COMPOSÉ A | 0,005 |
| DIPROPYLENE GLYCOL CARE | Dipropylene glycol | 40,000 |
| ETHANOL 95-96% | Ethanol | 20,000 |
| SIMULGEL 600 PHA | Acrylamide, AMPS Copolymer Dispersion 40%/ Isohexadecane/ polysorbate 80 | 3,000 |
| ST-CYCLOMETHICONE 5NF | Cyclopentasiloxane | 2,000 |
| EAU PURIFIEE | Purified water | QSP 100.000 |

### Formule n°7:

| **DENOMINATION COMMERCIALE** | **DENOMINATION INCI** | **%** |
|---|---|---|
| COMPOSÉ A | COMPOSÉ A | 0,005 |
| PROPYLENE GLYCOL | Propylene glycol | 40,000 |
| ETHANOL 95-96% | Ethanol | 20,000 |
| RONACARE ALLANTOIN | Allantoin | 0,200 |
| SIMULGEL 600 PHA | Acrylamide, AMPS Copolymer Dispersion 40%/ Isohexadecane/ polysorbate 80 | 3,000 |
| PHENOXETOL | Phenoxyethanol | 1,000 |
| EAU PURIFIEE | Purified water | QSP 100.000 |

### Formule n°8:

| **DENOMINATION COMMERCIALE** | **DENOMINATION INCI** | **%** |
|---|---|---|
| COMPOSÉ A | COMPOSÉ A | 0,010 |
| DIPROPYLENE GLYCOL CARE | Dipropylene glycol | 40,000 |
| ETHANOL 95-96% | Ethanol | 20,000 |
| RONACARE ALLANTOIN | Allantoin | 0,200 |
| SIMULGEL 600 PHA | Acrylamide, AMPS Copolymer Dispersion 40%/ Isohexadecane/ polysorbate 80 | 4,000 |
| ST-CYCLOMETHICONE 5NF | Cyclopentasiloxane | 1,000 |
| EAU PURIFIEE | Purified water | QSP 100.000 |

### Formule n°9:

| **DENOMINATION COMMERCIALE** | **DENOMINATION INCI** | **%** |
|---|---|---|
| COMPOSÉ A | COMPOSÉ A | 0,01 |
| DIPROPYLENE GLYCOL CARE | Dipropylene glycol | 30,000 |
| ETHANOL 95-96% | Ethanol | 15,000 |
| RONACARE ALLANTOIN | Allantoin | 0,200 |
| SIMULGEL 600 PHA | Acrylamide, AMPS Copolymer Dispersion 40%/ Isohexadecane/ polysorbate 80 | 3,000 |
| ST-CYCLOMETHICONE 5NF | Cyclopentasiloxane | 1,000 |
| EAU PURIFIEE | Purified water | QSP 100.000 |

### Exemple 3 : Caractérisation des formulations par des études de pénétration cutanée sur peau humaine:

Les études de pénétration cutanée permettent de caractériser les formulations, et de mettre en évidence des paramètres propres à chacune des formulations.

Deux types d'études de pénétration cutanée sur peau humaine ex vivo ont été réalisés.

Dans ces études le composé A correspond à l'acide 3"-tert-butyl-4'-(2-hydroxy-ethoxy)-4"-pyrrolidin-1-yl-[1,1';3',1"]-terphenyl-4-carboxylique.

Le gel de référence étant décrit comme suivant :

| **Constituants** | **%** |
|---|---|
| Composé A | 0.01 |
| Propylène Glycol | 30.00 |
| Ethanol 95-96% | 67.99 |
| Klucel HF Pharma | 2.00 |

Dans ces 2 études, la composition « gel optimisé » correspondant à la formule 3 est évaluée en comparaison à une composition « gel de référence ».

### 1- Etude de pénétration cutanée « temps unique »:

Dans cette étude, la formule est appliquée pendant 16h à la surface de la peau. A la fin de l'application, le composé A est quantifié dans les différents compartiments de la peau : stratum corneum, épiderme, derme et liquide récepteur selon une méthode de bioanalyse validée.

Les détails de l'application cutanée sont donnés dans le tableau ci-dessous.

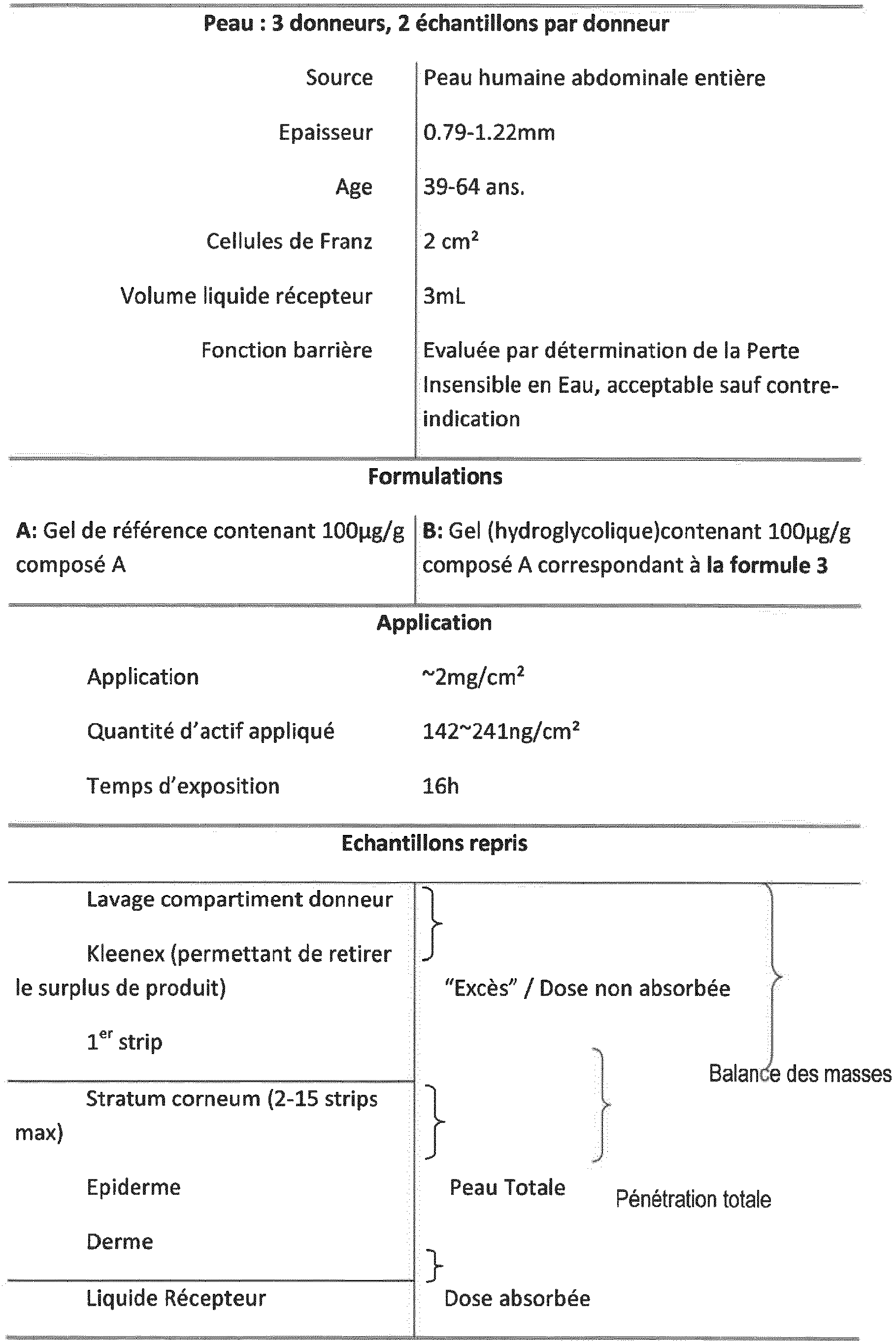

La bioanalyse a été réalisée par spectrométrie de masse en tandem par ionisation électrospray positive, et utilisant un appareil Xevo (Waters). La limite de quantification pour le composé A est de 1ng/mL.

Les conditions de LC/MS/MS mises au point ont permis de détecter jusqu'à 0,1% de la dose appliquée dans chacun des compartiments (dose non absorbée, stratum, épiderme, derme et liquide récepteur).

Les conditions techniques sont données dans le tableau ci-dessous.

| | | | | | | | |
|---|---|---|---|---|---|---|---|
| Colonne LC | Hypersil gold 50*2.1mm (UPLC) | | | | | | |
| Phase Mobile | Phase A : ACN + 0.1% Formic Acid | | | | | | |
| | Phase B : H₂O + 0.1% Formic acid | | | | | | |
| Lavage aiguille | ACN | | | | | | |
| Lavage septum | ACN/H₂O 50:50 | | | | | | |
| Gradient | Temps(min) | débit | %A | %B | Courbe | | |
| | 1. Initial | 0.700 | 15.0 | 85.0 | 0 | | |
| | 2.2.5 | 0.700 | 90.0 | 10.0 | 6 | | |
| | 3. 3.20 | 0.700 | 90.0 | 10.0 | 6 | | |
| | 4. 3.25 | 0.700 | 15.0 | 85.0 | 6 | | |
| Température de colonnes | | | | | | | |
| Détection MSMS | ESI+ MRM (E Electrosp ray Positif) | | | | | | |
| | Canal Réaction | | Dwell (secs) | Voltage (cône) | Col. Energy | Tr (min) | Composé |
| | 1: 460.26 > 318.20 | | 0.100 | 50.0 | 40.0 | 1.58 | Composé |
| | 1: 464.06 > 372.10 | | 0.100 | 55.0 | 40.0 | 1.58 | Standard |
| Volume Injection | 5µL | | | | | | |
| Temps de run | 4 minutes | | | | | | |

### a- Profil de distribution dans les différents compartiments (données qualitatives) :

La distribution entre les différents compartiments est du même type pour les 2 formules évaluées : accumulation dans le stratum corneum et taux de pénétration plus faible dans l'épiderme. Le composé A n'est pas détecté dans le liquide récepteur et est très faiblement présent dans le derme.

### b-Valeurs de pénétration dans le compartiment épiderme + derme :

Les valeurs de pénétration pour le gel optimisé (Exemple 3) contenant 100µg/g (0.01%) de composé A sont aux alentours de 9 ng/cm². Les niveaux de pénétration du composé A après application du gel optimisé (Exemple 3) tendent à être plus élevés que ceux obtenus après application du gel de référence.

### 1- Etude de cinétique de pénétration - Figure 1 :

Dans ce type d'étude, la pénétration de l'actif est quantifiée dans chaque compartiment de la peau après 0.5h, 1h, 3h 6h et 24h d'application. Une cinétique de pénétration dans chaque compartiment est alors déterminée et caractérisée.

Les détails de l'application cutanée sont donnés dans le tableau ci-dessous :

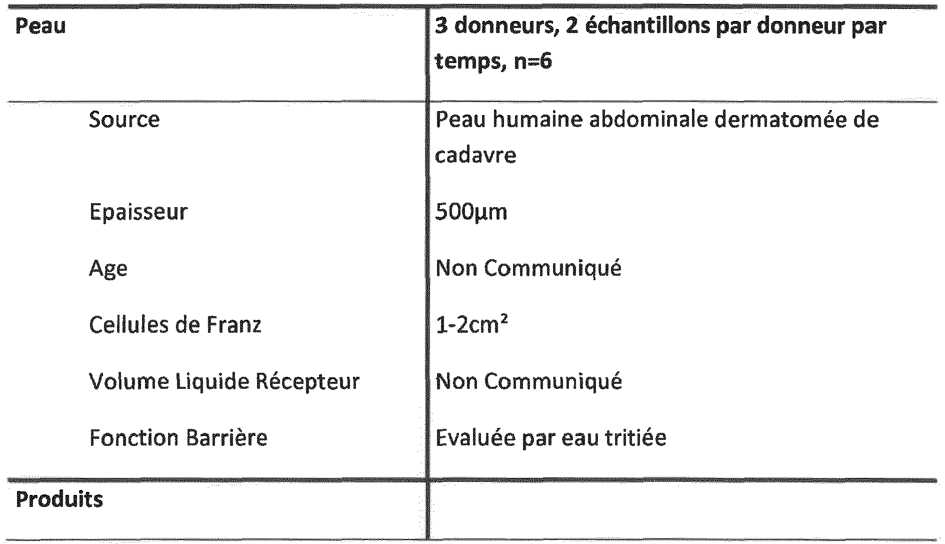

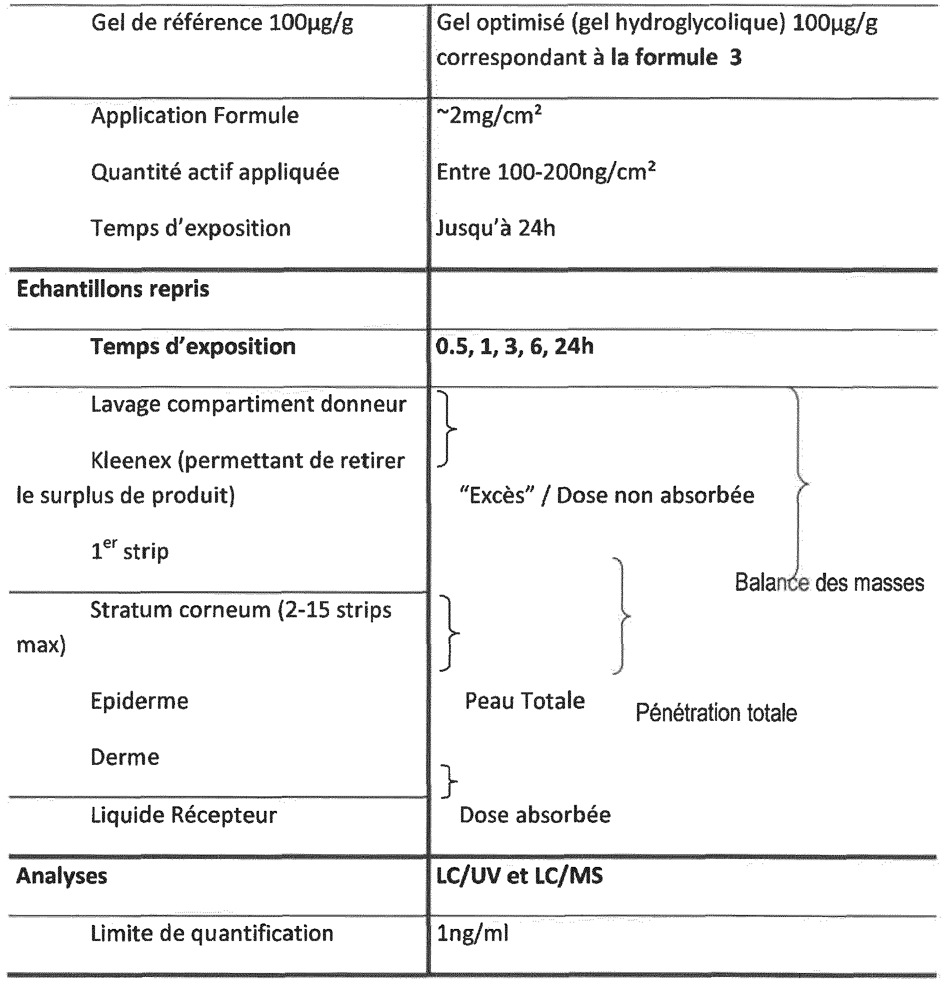

La quantité d'actif dans chaque compartiment à chaque temps a été déterminée par LC/UV ou par LC/MS. La méthode de bioanalyse a été validée de sorte à détecter au minimum 0,1% de la dose appliquée dans chaque compartiment.

Dans ce type d'étude, les paramètres retenus sont :
a. Le profil de la cinétique de pénétration dans l'épiderme (données qualitatives)
b. La vitesse initiale de la pénétration dans l'épiderme
c. La quantité maximale pénétrée dans l'épiderme

### a. Profil de la cinétique de pénétration dans l'épiderme - Figure 2 :

La cinétique de libération du composé A obtenue pour le gel optimisé (Exemple 3) présente une pente initiale élevée, suivie d'un plafond pendant lequel la pénétration du composé A n'augmente plus au cours du temps. La formule de référence (gel) montre le même profil avec une libération rapide au cours des premières heures et ensuite atteinte d'un plateau.

Comme vu dans le paragraphe 1 (Etude de pénétration cutanée « temps unique »), ces deux formules ont des niveaux de pénétration à 16h qui sont différents, la pénétration du composé A dans l'épiderme après application du gel optimisé (Exemple 3) tend à être plus élevée que celle obtenue après application du gel de référence.

### b. Vitesse initiale de la cinétique :

La valeur de vitesse initiale de la cinétique ou pente sur les 3 premières heures est de 4.12ng/cm²/h.

### c. Quantité maximale dans l'épiderme :

La quantité maximale dans l'épiderme est de 12.07ng/cm².

### Exemple 4 : Etude cosmétique d'amélioration du toucher :

### Essais pour amélioration du toucher:

Afin d'obtenir un toucher acceptable pour l'acné (non gras, non collant) tout en gardant une bonne solubilité du composé A dans la formule, des essais d'introduction de ST-cyclométhicone 5 ont été effectués sachant que la ST-cyclométhicone 5NF est un non-solvant du composé A et que cet ajout peut donc provoquer la recristallisation du composé A:

| ST-Cyclomethicone 5NF | 0% | 2% | 5% |
|---|---|---|---|
| Toucher | Toucher collant | Toucher acceptable | Toucher acceptable |
| Re-cristallisation du COMPOSÉ A | Pas de recristallisation | Pas de recristallisation | Recristallisation |

Ces essais nous ont permis d'optimiser la quantité de ST+cyclomethicone 5NF à 2% pour obtenir un toucher acceptable d'un point de vue cosmétique.

## Revendications

1. Composition pharmaceutique comprenant au moins un composé, **caractérisée en ce que** le composé est l'acide 3"-tert-butyl-4'-(2-hydroxy-ethoxy)-4"-pyrrolidin-1-yl-[1,1';3',1"]-terphenyl-4-carboxylique de formule generale (I) 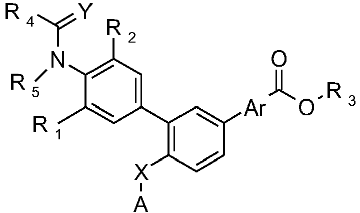 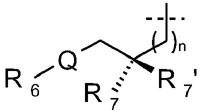 de l'eau, au moins un agent gélifiant, au moins un solvant hydrophile de l'acide 3"-tert-butyl-4'-(2-hydroxy-ethoxy)-4"-pyrrolidin-1-yl[1,1';3',1"]-terphenyl-4-carboxylique choisi parmi le phénoxyéthanol, l'éthanol et un mélange de phénoxyéthanol et d'éthanol, et au moins un co-solvant hydrophile de l'acide 3"-tert-butyl-4'-(2-hydroxy-ethoxy)-4"-pyrrolidin-1-yl-[1,1';3',1"]-terphenyl-4-carboxylique, **caractérisée en ce que** le co-solvant est choisi parmi le propylène glycol-, le dipropylène glycol et leurs mélanges.

2. Composition selon la revendication 1, **caractérisée en ce que** la composition est un gel hydroglycolique dans lequel l'acide 3"-tert-butyl-4'-(2-hydroxy-ethoxy)-4"-pyrrolidin-1-yl-[1,1';3',1"1-terphenyl-4-carboxylique est solubilisé.

3. Composition selon l'une quelconque des revendications précédentes, **caractérisée en ce que** l'agent gélifiant est choisi parmi : les polymères d'origine végétale, les gommes, les pectines, la cellulose et ses dérivés, les polymères d'origine microbiologiques tel que la gomme xanthane, et les polymères gélifiants d'origine synthétique,
et de préférence parmi:
- les Acrylates/C10-30 Alkyl Acrylate Crosspolymer vendu sous le nom de Pemulen TR-1 ou Pemulen TR-2
- les gélifiants de la famille des polyacrylamides comme le mélange Sodium acrylamide/acryloyldimethyltaurate copolymer / isohexadecane / polysorbate 80 vendu sous le nom de Simulgel 600PHA
- le mélange polyacrylamide/isoparaffine C13-14/laureth-7 vendu sous le nom de Sepigel 305,
- les carbomers vendus sous le nom d'Ultrez 20^{®}, d'Ultrez 10^{®}, de Carbopol 1382^{®} ou de Carbopol ETD2020NF^{®}, de carbopol 981 ou encore carbopol 980,
- les polysaccharides avec à titre d'exemples non limitatifs la gomme de xanthane telle que le Xantural180^{®}, la gellan gum vendu sous le nom de Kelcogel, la gomme guar, la cellulose et ses dérivés tel que la microcrystalline cellulose et carboxymethyl cellulose de sodium vendue sous le nom d'Avicel CL-611, l'hydroxypropylmethylcellulose en particulier le produit vendu sous le nom de Methocel E4M premium ou l'hydroxyéthylcellulose , en particulier, le produit vendu sous le nom de Natrosol HHX 250^{®} , le sodium carboxymethylcellulose en particulier la Blanose cellulose gum 7F,
- la famille des aluminium magnésium silicates tel que le Veegum K,
- la famille des polymères acryliques couplés à des chaînes hydrophobes tel que le PEG-150/decyl/SMDI copolymer vendu sous le nom de Aculyn 44,
- la famille des amidons modifiés tels que l'amidon de pomme de terre modifié vendu sous le nom de Structure Solanace ou bien leurs mélanges,
- la famille des carraghénanes en particulier réparties sous quatre grandes familles : κ, λ, β, ω tel que les Viscarin^{®} et les Gelcarin^{®}.

4. Composition selon l'une quelconque des revendications précédentes, **caractérisée** en ce en ce qu'elle contient en outre un ou des additifs choisis parmi :
- un ou plusieurs agents conservateurs, tels le méthyl parabène, le propyl parabène, le chlorure de benzalkonium, le phénoxyéthanol, l'alcool benzylique, le sorbate de potassium, l'acide benzoique, le 2-Bromo-2-Nitropropane-1,3-Diol, la chlorhexidine, le chlorocrésol et ses dérivés, l'alcool éthylique ou la diazolidinylurée.
- un ou plusieurs agents permettant d'améliorer les propriétés des formules à l'application comme la Cyclomethicone (St-Cyclomethicone 5NF) ou la Dimethicone (Q7 9120 silicon fluid de viscosité de 20 cst à 12500 cst)
- un ou plusieurs agent chélatant comme l'EDTA (ethylenediaminetetraacetic acid) et ses dérivés ou sels, la dihydroglycerine, les acides citriques et tartriques ou la gluconolactone.
- un ou plusieurs antioxydants tel que la vitamine E et ses dérivés, comme le DL alpha Tocopherol ou l'acetate de tocopherol, la vitamine C et ses dérivés, comme l'Ascorbyl Palmitate de Roche ou le Butylhydroxytoluene,
- un ou plusieurs agents apaisants et/ou anti-irritants tels que le PPG-12/SMDI copolymer, l'acide glycyrrhetinique ou ses dérivés, l'acide hyaluronique ou sa forme hyalurate de sodium ou l'allantoine.

5. Composition selon l'une des revendications 1 à 4 **caractérisée en ce qu'**elle comprend les ingrédients suivants :
- de 0,00001% à 1% en poids, préférentiellement de 0.0001 à 0.1 % en poids, et de manière plus préférée de 0,001 à 0.1 %en poids d'acide 3"-tert-butyl-4'-(2-hydroxy-ethoxy)-4"-pyrrolidin-1-yl-[1,1';3',1"]-terphenyl-4-carboxylique,
- de l'eau,
- de 0,005 à 10 % en poids, et préférentiellement de 1 à 4% en poids d'agent gélifiant,
- de 0.2 à 50% en poids, et préférentiellement de 0,5 à 30% en poids de solvant hydrophile,
- de 2 à 50% en poids, et préférentiellement de 10 à 40% en poids de co-solvant, et
- en option de 0 à 15% en poids, et préférentiellement de 0.1 à 10% en poids d'additifs.

6. Composition selon la revendication 1 **caractérisée en ce que** la quantité maximale de principe actif absorbée dans le derme et l'épiderme 16 heures après application est comprise entre 5 ng/cm² et 15 ng/cm.

7. Composition selon la revendication 1 **caractérisée en ce que** la quantité maximale de principe actif absorbée dans l'épiderme est obtenue entre 1 et 6 heures après application.

8. Composition selon l'une des revendications 1 à 7 pour son utilisation à titre de médicament.

9. Composition selon l'une des revendications 1 à 8 pour son utilisation dans le traitement d'une ou plusieurs des pathologies suivantes :
- Les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
- Les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
- Les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
- Les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
- Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
- Les désordres dermatologiques tels que les dermatoses immunes comme le lupus érythémateux, les maladies immunes bulleuses et les maladies du collagène, telle la sclérodermie ;
- Les stigmates de l'atrophie épidermique et/ou dermique induite par les corticostéroïdes locaux ou systémiques, ou toute autre forme d'atrophie cutanée,
- Les troubles de la cicatrisation, ou pour prévenir ou pour réparer les vergetures, ou encore pour favoriser la cicatrisation,
- Les affections d'origine fongiques au niveau cutané tel que le tinea pedis et le tinea versicolor,
- Les désordres de la pigmentation, tel l'hyperpigmentation, le mélasma, l'hypopigmentation ou le vitiligo ;
- Les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tel que le lymphome T.

## Patentansprüche

1. Pharmazeutische Zusammensetzung, umfassend zumindest eine Verbindung, **dadurch gekennzeichnet, dass** die Verbindung 3"-tert-Butyl-4'-(2-hydroxy-ethoxy)-4"-pyrrolidin-1-yl-[1,1';3',1"]-terphenyl-4-carbonsäure ist;
Wasser, zumindest ein Geliermittel, zumindest ein hydrophiles Lösungsmittel für 3"-tert-Butyl-4'-(2-hydroxy-ethoxy)-4"-pyrrolidin-1-yl-[1,1';3',1"]-terphenyl-4-carbonsäure, ausgewählt aus Phenoxyethanol, Ethanol und einer Mischung aus Phenoxyethanol und Ethanol, und zumindest ein hydrophiles Co-Lösungsmittel für 3"-tert-Butyl-4'-(2-hydroxy-ethoxy)-4"-pyrrolidin-1-yl-[1,1';3',1"]-terphenyl-4-carbonsäure, **dadurch gekennzeichnet, dass** das Co-Lösungsmittel aus Propylenglycol, Dipropylenglycol und Mischungen davon ausgewählt ist.

2. Zusammensetzung nach Anspruch 1, **dadurch gekennzeichnet, dass** die Zusammensetzung ein hydroglykolisches Gel ist, in dem 3"-tert-Butyl-4'-(2-hydroxy-ethoxy)-4"-pyrrolidin-1-yl-[1,1';3',1"]-terphenyl-4-carbonsäure aufgelöst ist.

3. Zusammensetzung nach einem der vorhergehenden Ansprüche, **dadurch gekennzeichnet, dass** das Geliermittel ausgewählt ist aus: Polymeren pflanzlichen Ursprungs, Gummis, Pektinen, Cellulose und Derivaten davon, Polymeren mikrobiologischen Ursprungs wie Xanthangummi, und gelierenden Polymeren synthetischen Ursprungs,
und bevorzugt aus:
- Acrylaten/C10-30-Alkylacrylat-Crosspolymer, vertrieben unter dem Namen Pemulen TR-1 oder Pemulen TR-2,
- Geliermitteln aus der Familie der Polyacrylamide wie die Mischung aus Natriumacrylamid/Acryloyldimethyltaurat-Copolymer/Isohexadecan/Polysorbat 80, vertrieben unter dem Namen Simulgel 600PHA,
- der Mischung Polyacrylamid/Isoparaffin C13-14/Laureth-7, vertrieben unter dem Namen Sepigel 305,
- Carbomeren, vertrieben unter den Namen Ultrez 20^{®}, Ultrez 10^{®}, Carbopol 1382^{®} oder Carbopol ETD2020NF^{®}, Carbopol 981 oder Carbopol 980,
- Polysacchariden mit, als nicht einschränkende Beispiele, Xanthangummi, wie Xantural180^{®}, Gellangummi, vertrieben unter dem Namen Kelcogel, Guargummi, Cellulose und Derivaten davon, wie mikrokristalline Cellulose und Natriumcarboxymethylcellulose, vertrieben unter dem Namen Avicel CL-611, Hydroxypropylmethylcellulose, insbesondere das Produkt, das unter dem Namen Methocel E4M Premium vertrieben wird, oder Hydroxyethylcellulose, insbesondere das Produkt, das unter dem Namen Natrosol HHX 250^{®} vertrieben wird, Natriumcarboxymethylcellulose, insbesondere Blanose Cellulose Gum 7F,
- der Familie der Aluminium-Magnesium-Silikate wie Veegum K,
- der Familie von Acrylpolymeren, die an hydrophobe Ketten gekoppelt sind, wie PEG-150/Decyl/SMDI-Copolymer, vertrieben unter dem Namen Aculyn 44,
- der Familie der modifizierten Stärken wie modifizierte Kartoffelstärke, vertrieben unter dem Namen Structure Solanace, oder Mischungen davon,
- der Familie der Carrageene, insbesondere unterteilt in vier Hauptfamilien: κ, λ, β, ω wie Viscarin^{®} und Gelcarin^{®}.

4. Zusammensetzung nach einem der vorhergehenden Ansprüche, **dadurch gekennzeichnet, dass** sie ferner einen oder mehrere Zusatzstoffe enthält, ausgewählt aus:
- einem oder mehreren Konservierungsmitteln, wie Methylparaben, Propylparaben, Benzalkoniumchlorid, Phenoxyethanol, Benzylalkohol, Kaliumsorbat, Benzoesäure, 2-Brom-2-Nitropropan-1,3-Diol, Chlorhexidin, Chlorcresol und Derivaten davon, Ethylalkohol oder Diazolidinylharnstoff,
- einem oder mehreren Mitteln, die ermöglichen, die Eigenschaften der Formeln bei Anwendung zu verbessern, wie Cyclomethicone (St-Cyclomethicone 5NF) oder Dimethicone (Q7 9120 Silikonflüssigkeit mit Viskosität von 20 cst bis 12500 cst),
- einem oder mehreren Chelatbildnern wie EDTA (Ethylendiamintetraessigsäure) und Derivaten oder Salzen davon, Dihydroglycerin, Zitronen- und Weinsäure oder Gluconolacton,
- einem oder mehreren Antioxidantien wie Vitamin E und Derivaten davon, wie DL alpha Tocopherol oder Tocopherolacetat, Vitamin C und Derivaten davon, wie Ascorbylpalmitat von Roche oder Butylhydroxytoluol,
- einem oder mehreren beruhigenden und/oder reizhemmenden Mitteln wie PPG-12/SMDI-Copolymer, Glycyrrhetinsäure oder Derivaten davon, Hyaluronsäure oder ihre Natriumhyaluratform oder Allantoin.

5. Zusammensetzung nach einem der Ansprüche 1 bis 4, **dadurch gekennzeichnet, dass** sie folgende Inhaltsstoffe umfasst:
- 0,00001 bis 1 Gew.-%, bevorzugt 0,0001 bis 0,1 Gew.-% und bevorzugter 0,001 bis 0,1 Gew.-% 3"-tert-Butyl-4'-(2-hydroxy-ethoxy)-4"-pyrrolidin-1-yl-[1,1';3',1"]-terphenyl-4-carbonsäure,
- Wasser,
- 0,005 bis 10 Gew.-% und bevorzugt 1 bis 4 Gew.-% Geliermittel,
- 0,2 bis 50 Gew.-% und bevorzugt 0,5 bis 30 Gew.-% hydrophiles Lösungsmittel,
- 2 bis 50 Gew.-% und bevorzugt 10 bis 40 Gew.-% Co-Lösungsmittel, und
- optional 0 bis 15 Gew.-% und bevorzugt 0,1 bis 10 Gew.-% Zusatzstoffe.

6. Zusammensetzung nach Anspruch 1, **dadurch gekennzeichnet, dass** die maximale Menge an Wirkstoff, die 16 Stunden nach Anwendung in die Dermis und die Epidermis absorbiert wird, zwischen 5 ng/cm² und 15 ng/cm² liegt.

7. Zusammensetzung nach Anspruch 1, **dadurch gekennzeichnet, dass** die maximale Menge an Wirkstoff, die in die Epidermis absorbiert wird, zwischen 1 und 6 Stunden nach Anwendung erhalten wird.

8. Zusammensetzung nach einem der Ansprüche 1 bis 7 für ihre Verwendung als Arzneimittel.

9. Zusammensetzung nach einem der Ansprüche 1 bis 8 für ihre Verwendung bei der Behandlung von einer oder mehreren der folgenden Pathologien:
- dermatologischen Erkrankungen im Zusammenhang mit einer Keratinisierungsstörung bezüglich Zelldifferenzierung und -proliferation, insbesondere zur Behandlung von Akne vulgaris, komedonaler Akne, polymorpher Akne, Rosazea, nodulozystischer Akne, Conglobata, Altersakne, sekundärer Akne wie Sonnen-, Arzneimittel- oder Berufsakne;
- Verhornungsstörungen, insbesondere Ichthyose, ichthyosiformen Zuständen, lamellärer Ichthyose, Morbus Darrier, palmoplantarer Keratodermie, Leukoplakie, Pityriasis rubra pilaris und leukoplasiformen Zuständen, Haut- oder Schleimhautflechten (bukkal);
- dermatologischen Erkrankungen mit einer entzündlichen immunallergischen Komponente, mit oder ohne Zellproliferationsstörung, und insbesondere allen Formen der Psoriasis, ob kutan, mukosal oder ungual, und sogar psoriatischer Arthritis oder sogar atopischer Dermatitis und der verschiedenen Formen von Ekzemen;
- Hauterkrankungen aufgrund einer Einwirkung von UV-Strahlung sowie zur Reparatur oder Bekämpfung der Hautalterung, ob lichtbedingt oder chronologisch, oder zur Verringerung von Pigmentierungen und aktinischen Keratosen oder jeglichen Pathologien, die mit chronologischer oder aktinischer Alterung verbunden sind, wie Xerose, Pigmentflecken und Falten;
- jeglichem Zustand im Zusammenhang mit gutartigen dermalen oder epidermalen Proliferationen, unabhängig davon, ob sie viralen Ursprungs sind oder nicht, wie gewöhnliche Warzen, flache Warzen, Molluscum contagiosum und Epidermodysplasia verruciformis, orale oder floride Papillomatose;
- dermatologischen Erkrankungen wie Immundermatosen wie Lupus erythematodes, bullösen Immunerkrankungen und Kollagenerkrankungen wie Sklerodermie;
- Stigmata der epidermalen und/oder dermalen Atrophie, die durch lokale oder systemische Kortikosteroide oder jegliche andere Form der Hautatrophie induziert werden,
- Heilungsstörungen oder zur Vorbeugung oder Reparatur von Dehnungsstreifen oder zur Förderung der Heilung,
- Pilzerkrankungen der Haut wie Tinea pedis und Tinea versicolor,
- Pigmentstörungen wie Hyperpigmentierung, Melasma, Hypopigmentierung oder Vitiligo;
- krebsartigen oder präkanzerösen Haut- oder Schleimhauterkrankungen wie aktinischen Keratosen, Morbus Bowen, Karzinomen in situ, Keratoakanthom und Hautkrebs wie Basalzellkarzinom (BCC), Plattenepithelkarzinom (SCC) und kutanen Lymphomen wie T-Zell-Lymphom.

## Claims

1. A pharmaceutical composition comprising at least one compound, **characterized in that** the compound is 3"-tert-butyl-4'-(2- hydroxyethoxy)-4"-pyrrolidin-1-yl[1,1';3',1"]terphenyl-4-carboxylic acid;
water, at least one gelling agent, at least one hydrophilic solvent of 3"-tert-butyl-4'-(2- hydroxyethoxy)-4"-pyrrolidin-1-yl[1,1';3',1"]terphenyl-4-carboxylic acid, chosen from phenoxyethanol, ethanol or a mixture of phenoxyethanol and ethanol, and at least one hydrophilic cosolvent of 3"-tert-butyl-4'-(2-hydroxyethoxy)-4"-pyrrolidin-1-yl[1,1';3',1"]terphenyl-4-carboxylic acid , **characterized in that** the cosolvent is chosen from propylene glycol, dipropylene glycol, and mixtures thereof.

2. The composition according to claim 1, **characterized in that** the composition is an aqueous-glycolic gel in which 3"-tert-butyl-4'-(2- hydroxyethoxy)-4"-pyrrolidin-1-yl[1,1';3',1"]terphenyl-4-carboxylic acid is solubilized.

3. The composition according to any one of the preceding claims, **characterized in that** the gelling agent is chosen from: polymers of vegetable origin, gums, pectins, cellulose and its derivatives, polymers of microbiological origin, such as xanthan gum, and gelling polymers of synthetic origin, and preferably from:
the acrylates/C10-30 alkyl acrylate crosspolymer sold under the name Pemulen TR-1 or Pemulen TR-2,
gelifiers of the polyacrylamide family, such as the sodium acrylamide/acryloyldimethyl taurate copolymer / isohexadecane / polysorbate 80 mixture sold under the name Simulgel 600PHA,
the polyacrylamide/isoparaffin C13-14/laureth-7 mixture sold under the name Sepigel 305,
the carbomers sold under the name Ultrez 20^{®}, Ultrez 10^{®}, Carbopol 1382^{®} or Carbopol ETD2020NF^{®}, carbopol 981 or else carbopol 980,
polysaccharides with, by way of nonlimiting examples, xanthan gum, such as Xanturall80^{®}, the gellan gum sold under the name Kelcogel, guar gum, cellulose and its derivatives, such as the microcrystalline cellulose and sodium carboxymethylcellulose sold under the name Avicel CL-611, hydroxypropylmethylcellulose, in particular the product sold under the name Methocel E4M premium, or hydroxyethylcellulose , in particular the product sold under the name Natrosol HHX 250^{®}, sodium carboxymethylcellulose, in particular Blanose cellulose gum 7F,
the family of aluminum magnesium silicates, such as Veegum K,
the family of acrylic polymers coupled to hydrophobic chains, such as the PEG- 150/decyl/SMDI copolymer sold under the name Aculyn 44,
the family of modified starches, such as the modified potato starch sold under the name Structure Solanace or else mixtures thereof,
the family of carrageenans, in particular divided up into four major families: k, *λ*, *β*, *ω*, such as the Viscarin^{®} products and the Gelcarin^{®} products.

4. The composition according to any one of the preceding claims, **characterized in that** it also contains one or more additives chosen from:
- one or more preserving agents, such as methyl paraben, propyl paraben, benzalkonium chloride, phenoxyethanol, benzyl alcohol, potassium sorbate, benzoic acid, 2-bromo-2-nitropropane-1,3-diol, chlorhexidine, chlorocresol and its derivatives, ethyl alcohol or diazolidinylurea,
- one or more agents for improving the properties of the formulae on application, such as cyclomethicone (St-Cyclomethicone 5NF) or dimethicone (Q7 9120 silicon fluid having a viscosity of 20 cst to 12500 cst),
- one or more chelating agents, such as EDTA (ethylenediaminetetraacetic acid) and its derivatives or salts, dihydroglycerol, citric and tartaric acids or gluconolactone,
- one or more antioxidants such as vitamin E and its derivatives, for instance DL-alpha-tocopherol or tocopheryl acetate, vitamin C and its derivatives, for instance ascorbyl palmitate from Roche, or butylhydroxytoluene,
- one or more soothing agents and/or anti-irritants, such as the PPG-12/SMDI copolymer, glycyrrhetinic acid or its derivatives, hyaluronic acid or its sodium hyaluronate form, or allantoin.

5. The composition according to one of claims 1 to 4, **characterized in that** it comprises the following ingredients:
- from 0.00001% to 1% by weight, preferentially from 0.0001% to 0.1% by weight, and more preferably from 0.001% to 0.1% by weight, of 3"-tert-butyl-4'-(2-hydroxyethoxy)-4"- pyrrolidin-1-yl[1 ,1';3',1 "]terphenyl-4-carboxylic acid,
- water,
- from 0.005% to 10% by weight and preferentially from 1% to 4% by weight of gelling agent,
- from 0.2% to 50% by weight and preferentially from 0.5% to 30% by weight of hydrophilic solvent,
- from 2% to 50% by weight and preferentially from 10% to 40% by weight of cosolvent, and
- optionally, from 0 to 15% by weight and preferentially from 0.1% to 10% by weight of additives.

6. The composition according to claim 1, **characterized in that** the maximum amount of active ingredient absorbed in the dermis and the epidermis 16 hours after application is between 5 ng/cm² and 15 ng/cm².

7. The composition according to claim 1, **characterized in that** the maximum amount of active ingredient absorbed in the epidermis is obtained between 1 and 6 hours after application.

8. The composition according to one of claims 1 to 7, for use as a medicament.

9. The composition according to one of claims 1 to 8, for use in the treatment of one or more of the following pathological conditions:
- dermatological conditions associated with a keratinization disorder relating to cell differentiation and proliferation, in particular for treating common acne, comedonal acne, polymorphic acne, acne rosacea, nodulocystic acne, acne conglobata, senile acne, secondary acne such as solar acne, acne medicamentosa or occupational acne;
- keratinization disorders, in particular ichthyosis, ichthyosiform conditions, lamellar ichthyosis, Darier's disease, palmoplantar keratoderma, leukoplakia, pityriasis rubra pilaris and leukoplakiform conditions, cutaneous or mucosal (buccal) lichen;
- dermatological conditions with an inflammatory immunoallergic component, with or without a cell proliferation disorder, and in particular all forms of psoriasis, whether cutaneous, mucosal or ungual, and even psoriatic arthritis, or else atopic dermatitis and the various forms of eczema;
- skin disorders caused by exposure to UV radiation, and also for repairing or combating skin aging, whether it is photo-induced or chronological, or for reducing actinic keratoses and pigmentations, or any pathological conditions associated with chronological or actinic aging, such as xerosis, pigmentations and wrinkles;
- any condition associated with benign dermal or epidermal proliferations, whether or not they are of viral origin, such as common warts, flat warts, molluscum contagiosum and epidermodysplasia verruciformis, or oral or florid papillomatoses;
- dermatological disorders such as immune dermatoses, for instance lupus erythematosus, bullous immune diseases and collagen diseases, such as scleroderma;
- stigmata of epidermal and/or dermal atrophy induced by local or systemic corticosteroids, or any other form of cutaneous atrophy;
- healing disorders, or for preventing or repairing stretch marks, or else for promoting healing;
- conditions of fungal origin at the cutaneous level, such as tinea pedis and tinea versicolor;
- pigmentation disorders, such as hyperpigmentation, melasma, hypopigmentation or vitiligo;
- cutaneous or mucosal cancerous or precancerous conditions, such as actinic keratoses, Bowen's disease, in-situ carcinomas, keratoacanthomas and skin cancers such as basal cell carcinoma (BCC), squamous cell carcinoma (SCC) and cutaneous lymphomas such as T lymphoma.
